(19) Europäisches Patentamt
European Patent Office
Office européen des brevets

(11) **EP 3 024 863 B1**

(12) **EUROPÄISCHE PATENTSCHRIFT**

(45) Veröffentlichungstag und Bekanntmachung des Hinweises auf die Patenterteilung:
**04.05.2022   Patentblatt 2022/18**

(21) Anmeldenummer: **14741867.7**

(22) Anmeldetag: **21.07.2014**

(51) Internationale Patentklassifikation (IPC):
**C08G 18/02** *(2006.01)*     **C08G 18/16** *(2006.01)*
**C08G 18/78** *(2006.01)*     **C08G 18/79** *(2006.01)*
**C07D 273/04** *(2006.01)*

(52) Gemeinsame Patentklassifikation (CPC):
**C07D 273/04; C08G 18/02; C08G 18/022; C08G 18/166; C08G 18/7887; C08G 18/79; C08G 18/792**

(86) Internationale Anmeldenummer:
**PCT/EP2014/065578**

(87) Internationale Veröffentlichungsnummer:
**WO 2015/011071 (29.01.2015 Gazette 2015/04)**

(54) **VERFAHREN ZUR HERSTELLUNG VON IMINOOXADIAZINDIONGRUPPEN ENTHALTENDEN POLYISOCYANATEN UND DEREN VERWENDUNG.**

METHOD FOR MANUFACTURING POLYISOCYANATES CONTAINING IMINOOXADIAZINDIONE GROUPS AND THEIR APPLICATION

PROCÉDÉ DE FABRICATION DE POLYISOCYANATES CONTENANT DES GROUPES IMINOOXADIAZINDIONE ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.07.2013   EP 13177981**

(43) Veröffentlichungstag der Anmeldung:
**01.06.2016   Patentblatt 2016/22**

(73) Patentinhaber: **Covestro Intellectual Property GmbH & Co. KG**
**51373 Leverkusen (DE)**

(72) Erfinder:
• **RICHTER, Frank**
**51373 Leverkusen (DE)**
• **HALPAAP, Reinhard**
**51519 Odenthal (DE)**

(74) Vertreter: **Levpat**
**c/o Covestro AG**
**Gebäude 4825**
**51365 Leverkusen (DE)**

(56) Entgegenhaltungen:
**EP-A1- 0 962 455**        **DE-A1-102004 048 871**
**DE-A1-102005 002 867**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von Iminooxadiazindiongruppen enthaltenden Polyisocyanaten, bei dem mindestens ein monomeres Di- und/oder Triisocyanat, in Gegenwart von

a) mindestens einem Katalysator,
b) mindestens einem Additiv (A) mit einer relativen Permittivität bei 18 °C bis 30°C von weniger als 4,0, bestimmt mit derjenigen Messmethode wie sie in der Beschreibung angegeben ist,
c) gegebenenfalls weiteren, von A verschiedenen Additiven

oligomerisiert wird, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ($[F^- \times HF]_m]$), wobei m einen Zahlenwert von 0,001 bis 2,000 besitzt. Die Erfindung betrifft ferner ein Reaktionssystem zur Herstellung von Iminooxadiazindiongruppen enthaltenden Polyisocyanaten sowie die Verwendung eines Additivs (A) mit einer relativen Permittivität bei 18 °C bis 30 °C von weniger als 4,0 zur Herstellung von Iminooxadiazindiongruppen enthaltenden Polyisocyanaten durch katalysierte Modifizierung monomerer Di- und/oder Triisocyanate.

Die Oligo- bzw. Polymerisierung von Isocyanaten, hier zusammenfassend Modifizierung genannt, ist seit langem bekannt. Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert oder abtrennt) und das erhaltene Polyisocyanat in der Regel vom nicht umgesetzten Monomer separiert. Eine Zusammenstellung dieser Verfahren des Standes der Technik findet sich in H. J. Laas et al., J. Prakt. Chem. 1994, 336, 185 ff.

Eine spezielle Form der Isocyanatmodifizierung die zu Produkten mit einem hohen Anteil an Iminooxadiazindiongruppen (asymmetrischen Isocyanat-Trimeren), neben den lange bekannten Isocyanuratstrukturen (symmetrischen Isocyanat-Trimeren, bisher vereinfachend häufig nur als "Trimere" bezeichnet) in den Verfahrensprodukten führt, wird u.a. in EP 962 455 A1, EP 962 454 A1, EP 896 009 A1, EP 798 299 A1, EP 447 074 A1, EP 379 914 A1, EP 339 396 A1, EP 315 692 A1, EP 295 926 A1 sowie EP 235 388 A1 beschrieben. Als Katalysatoren hierfür haben sich u.a. (Hydrogenpoly)fluoride bewährt.

Ein Nachteil der bekannten Verfahren des Standes der Technik ist, dass der Iminooxadiazindionanteil in den Verfahrensprodukten nur etwa 50%, bezogen auf die Summe aus symmetrischem (Isocyanurat) und asymmetrischem Trimer (Iminooxadiazindion) beträgt und dieser Anteil bei höherem Umsatz an Monomer weiter abnimmt.

Zwar lässt sich durch Erhöhung des "HF-Anteils" im Katalysator, d.h. bei Übergang von einfachen Fluoriden (die in der Regel nicht langzeitstabil sind und allmählich auch ohne externe HF-Zugabe in die Difluoridform übergehen) zu Difluoriden, Trifluoriden etc. der Iminooxadiazindionanteil in den Verfahrensprodukten in der gewünschten Richtung beeinflussen, jedoch hat diese Herangehensweise Nachteile (höherer HF-Anteil im Prozeßabgas, das aufwendig neutralisiert werden muss, höhere Korrosivität der Katalysatorlösungen etc.), die die Vorteile nicht aufwiegen.

EP 0 962 455 A1 offenbart die Trimerisierung von Hexamethylendiisocyanat (HDI) im Beisein der quaternären Phosphoniumfluoride $Bu_4P^+F^-$, bzw. $R_4P^+F^-$ als Katalysatorkomponenten, wobei der Anteil an Iminooxadiazindion-Struktur bis zu 64 mol-% im Trimergemisch betragen kann.

Der Erfindung lag daher die Aufgabe zugrunde, ein Verfahren zur Herstellung hoch Iminooxadiazindiongruppen enthaltender Polyisocyanate zur Verfügung zu stellen, das nicht mit den vorgenannten Nachteilen behaftet ist: die Verfahrensprodukte sollten auch ohne Erhöhung des "HF-Anteils" im Katalysator einen höheren Anteil an Iminooxadiazindionstrukturen aufweisen, als die Produkte, die nach bekannten Verfahren des Standes der Technik zugänglich sind.

Die Aufgabe wird gelöst durch ein Verfahren zur Herstellung von Iminooxadiazindiongruppen enthaltenden Polyisocyanaten, bei dem mindestens ein monomeres Di- und/oder Triisocyanat, in Gegenwart von

a) mindestens einem Katalysator,
b) mindestens einem Additiv (A) mit einer relativen Permittivität bei 18 °C bis 30 °C von weniger als 4,0, bestimmt mit derjenigen Messmethode wie sie in der Beschreibung angegeben ist,
c) gegebenenfalls weiteren, von A verschiedenen Additiven

oligomerisiert wird, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([F⁻ × HF)ₘ]), wobei m einen Zahlenwert von 0,001 bis 2,000 besitzt.

Die relative Permittivität bei 18 °C bis 30 °C wird im Sinne der vorliegenden Erfindung bestimmt durch das Verhältnis der Kapazitäten eines Kondensators mit der Substanz einerseits und Vakuum andererseits als Dielektrikum bei einer Messfrequenz von 50 Hz. Dies lässt sich durch den allgemein bekannten folgenden Zusammenhang ausdrücken:

$$\varepsilon_r = \frac{\varepsilon}{\varepsilon_0}$$

mit der relative Permittivität $\varepsilon_r$, gemessene Permittivität der Substanz $\varepsilon$ und zu der elektrischen Feldkonstante $\varepsilon_0$. Hierbei bedeutet der genannte Temperaturbereich von 18 °C bis 30 °C, dass die Substanz bei einer beliebigen Temperatur in diesem Bereich die angegebene relative Permittivität aufweist. Besitzt ein Additiv also beispielsweise bei 30 °C eine relative Permittivität von 3,0, bei 20 °C hingegen ein höhere relative Permittivität als beispielsweise 4,0, so erfüllt dieses Additiv dennoch die erfindungsgemäße Definition einer relativen Permittivität bei 18 °C bis 30 °C von weniger als 4,0. Selbiges gilt analog für die weiter unten definierten Vorzugsbereiche hinsichtlich der relativen Permittivität.

Die folgende Tabelle vermittelt einen Überblick über typische Werte der relativen Permittivitäten verschiedener Stoffe, jeweils gemessen bei 50 Hz:

| Lösungsmittel | relative Permittivität bei | °C |
|---|---|---|
| n-Hexan | 1,9 | 25 |
| n-Heptan | 2,0 | 25 |
| Cyclohexan | 2,0 | 20 |
| Isoeicosan | 2,1 | 25 |
| 1,4-Dioxan | 2,2 | 25 |
| Tetrachlorkohlenstoff | 2,2 | 20 |
| Benzol | 2,3 | 25 |
| Tetrachlorethen | 2,3 | 25 |
| Toluol | 2,4 | 25 |
| Triethylamin | 2,4 | 25 |
| Schwefelkohlenstoff | 2,6 | 20 |
| Trichlorethen | 3,4 | 16 |
| Anisol | 4,3 | 25 |
| Dibutylether | 4,3 | 20 |
| Diethylether | 4,3 | 20 |
| Chloroform | 4,8 | 20 |
| Brombenzol | 5,4 | 25 |
| Chlorbenzol | 5,6 | 25 |
| Piperidin | 5,8 | 20 |
| Essigsäureethylester | 6,0 | 25 |
| Eisessig | 6,2 | 20 |
| Anilin | 6,9 | 20 |
| Ethylenglycoldimethylether | 7,2 | 25 |
| Triethylenglycoldimethylether (Triglyme) | 7,5 | 25 |
| 1,1,1-Trichlorethan | 7,5 | 20 |
| Tetrahydrofuran | 7,6 | 25 |

(fortgesetzt)

| Lösungsmittel | relative Permittivität bei | °C |
|---|---|---|
| Diethylenglycol | 7,7 | 25 |
| Methylenchlorid | 8,9 | 25 |
| Chinolin | 9,0 | 25 |
| Ethylendichlorid | 10,4 | 25 |
| Pyridin | 12,4 | 21 |
| 2-Methyl-2-propanol (tert-Butanol) | 12,5 | 25 |
| 3-Methyl-1-butanol (Isoamylalkohol) | 14,7 | 25 |
| 1-Butanol | 17,5 | 25 |
| Methylethylketon (Butanon) | 18,5 | 20 |
| 2-Propanol (Isopropylalkohol) | 19,9 | 25 |
| Propanol | 20,3 | 25 |
| Essigsäureanhydrid | 20,7 | 19 |
| Aceton | 20,7 | 25 |
| Triethylenglycol | 23,7 | 20 |
| Ethanol | 24,6 | 25 |
| Benzonitril | 25,2 | 25 |
| Adiponitril | 30,0 | 18 |
| N-Methyl-2-pyrrolidon (NMP) | 32,2 | 25 |
| Methanol | 32,7 | 25 |
| Nitrobenzol | 34,8 | 25 |
| Nitromethan | 35,9 | 30 |
| Gamma-Valerolacton | 36,9 | 20 |
| Dimethylformamid | 37,0 | 25 |
| Acetonitril | 37,5 | 20 |
| Ethylenglycol | 37,7 | 25 |
| Dimethylacetamid | 37,8 | 25 |
| $\gamma$-Butyrolacton | 39,1 | 25 |
| Sulfolan | 43,3 | 30 |
| Dimethylsulfoxid | 46,7 | 25 |
| Propylencarbonat (4-Methyl-1,3-dioxol-2-on) | 65,1 | 25 |
| Wasser | 78,4 | 25 |
| Formamid | 111,0 | 20 |
| N-Methylformamid | 182,4 | 25 |

Im Rahmen des erfindungsgemäßen Verfahrens kann als Additiv (A) ein lineares oder verzweigtes, aliphatisches, cycloaliphatisches und/oder araliphatisches $C_4$-$C_{30}$-Alkan, insbesondere $C_5$-$C_{30}$-Alkan, oder Mischungen davon eingesetzt werden. So ist das Additiv (A) insbesondere ausgewählt ist aus der Gruppe umfassend lineares, verzweigtes oder cyclisches Butan, Pentan, Hexan, Heptan, Octan, Nonan, Decan, Undecan, Dodecan, Tridecan, Tetradecan, Pentadecan, Hexadecan, Heptadecan, Octadecan, Nonadecan, Eicosan, Heneicosan, Docosan, Tricosan, -Tetracosan, Pentacosan, Hexacosan, Heptacosan, Octacosan, Nonacosan, Tricontan, sowie deren Mischungen.

Keiner der eingangs genannten Schriften des Standes der Technik ist zu entnehmen, dass die zur Iminooxadiazindionbildung bevorzugten Katalysatoren des Standes der Technik in Gegenwart der vorgenannten Additive eine signifikante Erhöhung des Iminooxadiazindionanteils in den Verfahrensprodukten, insbesondere bei Erhöhung des Monomerumsatzes, bewirken.

In vorteilhafter Ausgestaltung des erfindungsgemäßen Verfahrens weist das Additiv (A) eine relative Permittivität bei 18 °C bis 30 °C von höchstens 3,5 auf, bevorzugt von höchstens 3,0, besonders bevorzugt von höchstens 2,8 oder gar höchstens 2,5.

Die Menge an einzusetzendem Additiv kann im erfindungsgemäßen Verfahren in breiten Grenzen variieren. Bevorzugt liegt sie von 1 bis 50 Gew.-% bezogen auf die Masse des zu modifizierenden monomeren Di- und/oder Triisocyanats, besonders bevorzugt von 2 bis Gew.-30%, ganz besonders bevorzugt von 2 bis 20 Gew.-% . Technisch vorteilhaft ist natürlich eine möglichst niedrige Additivmenge um einerseits die Raum-Zeit-Ausbeute an Polyisocyanatharz hoch und den Katalysatorbedarf niedrig zu gestalten. Aber selbst bei Zusatz von 20% Isoeicosan liegt der Mehrbedarf an Katalysator noch durchaus im technisch akzeptablen Bereich bei deutlich erhöhtem - und vor allem auch bei höherem Monomerumsatz nicht so stark wie ohne Additiv abfallenden - Iminooxadiazindionanteil in den resultierenden Polyisocyanatharzen.

Mit dem erfindungsgemäßen Modifizierverfahren ist daher eine verbesserte Methode zur Herstellung hoch Iminooxadiazindiongruppen enthaltender Polyisocyanate in einfacher Weise zugänglich geworden.

In einer Ausführungsform des erfindungsgemäßen Verfahrens wird das Additiv (A) dem/den zu modifizierenden Monomeren beigemischt. In weiterer Ausgestaltung des erfindungsgemäßen Verfahrens wird dem monomeren Di- und/oder Triisocyanat das Additiv (A) vor dem Inkontaktbringen mit dem Katalysator zugesetzt.

Im Rahmen des erfindungsgemäßen Verfahrens können im Prinzip alle bekannten Isocyanate eingesetzt werden. Bevorzugt werden Di- und/oder Triisocyanate mit aliphatisch, cycloaliphatisch, araliphatisch und/oder aromatisch gebundenen Isocyanatgruppen einzeln oder in beliebigen Abmischungen untereinander eingesetzt. Hierbei ist es belanglos, nach welchen Verfahren die vorstehend genannten (Poly)isocyanate generiert werden, d.h. mit oder ohne Verwendung von Phosgen. Insbesondere seien genannt: Hexamethylendiisocyanat (HDI), 2-Methylpentan-1,5-diisocyanat, 2,4,4-Tiimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Poymer-MDI). Bevorzugt werden aliphatische Di- und/oder Triisocyanate, besonders bevorzugt aliphatische Diisocyanate eingesetzt. Ganz besonders bevorzugt wird Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat und/oder 4-Isocyanatomethyl-1,8-octandiisocyanat eingesetzt, noch mehr bevorzugt ist HDI.

Als Katalysatoren kommen prinzipiell alle für diesen Zweck vorbeschriebenen Verbindungen des Standes der Technik als solche oder in Lösung in Frage. Besonders geeignet sind salzartig aufgebaute Substanzen mit Kationen, die für eine gute Löslichkeit im Isocyanatmedium sorgen. Bevorzugt besitzt bei den Di- und/oder Poly(hydrogen)fluoriden ($[F^- \times HF)_m]$) m einen Zahlenwert von 0,1 bis 2,000, bevorzugt 0,5 bis 2,000.

Bei dem Di- und/oder Poly(hydrogen)fluorid ($[F^- \times HF)_m]$) kann es sich insbesondere um ein quaternäres Ammoniumdifluoridund/oder ein Phosphoniumdifluorid handelt, , vorzugsweise um solche, wie sie durch Abmischen von quaternären Ammonium- sowie Phosphonium-fluoriden oder -hydroxiden mit entsprechenden Mengen, optional in Alkoholen oder Wasser vorgelöstem Fluorwasserstoff bereitet werden können.

Als Lösungsmittel für den/die Katalysatoren kommen alle Verbindungen in Frage, die nicht mit dem Katalysator reagieren und ihn in ausreichendem Maße zu lösen vermögen. Dies sind z.B. für die o.g. Tetraorganyl-ammoniumsalze und -phosphoniumsalze aliphatische oder aromatische Kohlenwasserstoffe, Alkohole, Ester sowie Ether. Bevorzugt werden Alkohole verwendet.

Der Katalysatorbedarf im erfindungsgemäßen Verfahren unterscheidet sich dabei nicht signifikant von dem in der Bulk-Modifizierung des Standes der Technik beobachteten. Der Katalysator kann beispielweise in einem Anteil von 1 mol-ppm bis 1 mol-%, bevorzugt 5 mol-ppm und 0,1 mol-%, bezogen auf die Monomermenge, eingesetzt werden.

Das erfindungsgemäße Verfahren kann beispielsweise im Temperaturbereich von 0 °C bis + 250 °C durchgeführt werden, bevorzugt 20 bis 180 °C, besonders bevorzugt 40 bis 150 °C.

In weiterer Ausgestaltung des erfindungsgemäßen Verfahrens kann die Oligomerisierung abgebrochen werden, nachdem 5 bis 80 Gew.-% des eingesetzten monomeren Di- und/oder Triisocyanates umgesetzt sind, bevorzugt 10 bis 60 Gew.-%. Die Oligomerisierung kann beispielsweise abgebrochen werden, indem der Katalysator desaktiviert wird. Zur Katalysatordeaktivierung bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration oder Kombinationen hiervon.

Im Anschluss an die Katalysatordesaktivierung kann das nicht umgesetzte Monomer sowie gegebenenfalls mitverwendetes Lösungsmittel mit Hilfe aller bekannten Separationstechniken wie z.B. Destillation, gegebenenfalls in der speziellen Ausführungsform der Dünnschichtdestillation Extraktion oder Kristallisation/Filtration abgetrennt werden. Selbstverständlich können auch Kombinationen zweier oder mehrerer dieser Techniken angewendet werden.

Soll das erfindungsgemäß hergestellte Polyisocyanat noch freies, nicht umgesetztes Monomer enthalten, wie es z.B. für die Weiterverarbeitung zu NCO-blockierten Produkten von Interesse ist, so kann nach Katalysatordesaktivierung auf die Monomerenabtrennung verzichtet werden.

Bevorzugt wird das nicht umgesetzte Monomer abgetrennt, insbesondere destillativ. Bevorzugt weisen die erfindungsgemäßen Produkte nach der Abtrennung einen Restmonomergehalt < 0,5 Gew.-% auf, bevorzugt < 0,25 Gew.- %, besonders bevorzugt < 0,1 Gew.- %.

Verglichen mit der Katalyse z.B. durch quaternäre Phosphoniumsalze ohne Verwendung von Additiven (Bulk-Modifizierung, s. Vergleichsbeispiel 1), beobachtet man im erfindungsgemäßen Verfahren bei sonst gleichen Reaktionsbedingungen eine deutliche Erhöhung des Iminooxadiazindionanteils in den Verfahrensprodukten, insbesondere bei höherem Monomerumsatz.

Nach einer weiter bevorzugten, kontinuierlich arbeitenden Ausführungsform des erfindungsgemäßen Verfahrens kann die Oligomerisierung in einem Rohrreaktor vorgenommen werden. Dies ist deshalb vorteilhaft, da sich hierbei eine signifikant geringere Neigung der erfindungsgemäßen Katalysatoren spontan Gelteilchen im Produkt zu bilden im Vergleich zu den bekannten Katalysatoren des Standes der Technik ergibt, selbst bei Applikation in hochkonzentrierter Lösung bzw. als reiner Wirkstoff.

Die vorliegende Erfindung betrifft ferner ein Reaktionssystem zur Herstellung von Iminooxadiazindiongruppen enthaltenden Polyisocyanaten, umfassend mindestens ein monomeres Di- und/oder Triisocyanat, sowie

a) mindestens einem Katalysator,

b) mindestens ein Additiv (A) mit einer relativen Permittivität bei 18 °C bis 30 °C von weniger als 4,0, bestimmt mit derjenigen Messmethode wie sie in der Beschreibung angegeben ist, insbesondere von höchstens 3,5, bevorzugt von höchstens 3,0, besonders bevorzugt von höchstens 2,8 oder gar höchstens 2,5,

c) gegebenenfalls weitere, von (A) verschiedene Additive,

wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([$F^-$ × $HF)_m$]), wobei m einen Zahlenwert von 0,001 bis 2,000 besitzt.

Ein weiterer Gegenstand der vorliegenden Erfindung betrifft die Verwendung von Verbindungen mit einer relativen Permittivität bei 18 °C bis 30 °C von weniger als 4,0, bestimmt mit derjenigen Messmethode wie sie in der Beschreibung angegeben ist, insbesondere von höchstens 3,5, bevorzugt von höchstens 3,0, besonders bevorzugt von höchstens 2,8 oder gar höchstens 2,5 als Additiv (A) zur Herstellung von Iminooxadiazindiongruppen enthaltenden Polyisocyanaten durch katalysierte Modifizierung monomerer Di- und/oder Triisocyanate, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([$F^-$ × $HF)_m$]), wobei m einen Zahlenwert von 0,001 bis 2,000 besitzt.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Insbesondere zur Herstellung von, ggf. wasserdipergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte auf Isocyanuratbasis.

Sie können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

**Beispiele**

Die vorliegende Erfindung wird im Folgenden anhand von Beispielen näher erläutert.

Alle Mengenangaben beziehen sich, soweit nicht anders vermerkt, auf die Masse.

Die Ermittlung des NCO-Gehaltes der in den Beispielen und Vergleichsbeispielen beschriebenen Harze erfolgte durch Titration gemäß DIN 53 185.

Der Phosphorgehalt aller Proben wurde durch Röntgenfluoreszenzanalyse (RFA) bestimmt.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen ($^1$H-NMR) bzw. ca. 50 %igen ($^{13}$C-NMR) Proben in trockenem $C_6D_6$ bei einer Frequenz von 400 bzw. 700 MHz ($^1$H-NMR) oder 100 bzw. 176 MHz ($^{13}$C-NMR). Als Referenz für die ppm-Skale wurden geringe Mengen von Tetramethylsilan im Lösungsmittel mit 0 ppm $^1$H-NMR-chem. Verschiebung heran gezogen. Alternativ wurde auf das Signal des im Lösungsmittel enthaltenen $C_6D_5H$ referenziert: 7,15 ppm $^1$H-NMR-chem. Verschiebung, 128,02 ppm $^{13}$C-NMR-chem. Verschiebung.. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP-A 896 009.

Die Bestimmung der Restmonomergehalte erfolgte gaschromatographisch.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen. Die Herstellung der Hydrogenpolyfluoridkatalysatoren ist literaturbekannt und wird u.a. in EP 962 454 beschrieben. Das verwendete Isoeicosan ist ein Isomerengemisch verschiedener C20-Alkane und wurde von der Fa. Ineos, D-50769, Köln bezogen.

**Beispiel 1 Vergleichsbeispiel**

In einem doppelwandigen, durch einen externen Kreislauf auf 60°C temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurde bei 20°C der Brechungsindex bei der Frequenz des Lichtes der D-Linie des Na-Emissionsspektrums (im weiteren Text $n_D^{20}$) gemessen und anschließend die in Tabelle 1 angegebene Katalysatormenge (bezogen auf die Masse an eingesetztem HDI, als 70%ige Lösung in Isopropanol) portionsweise so dosiert, dass die Innentemperatur 65°C nicht überstieg. Nachdem ca. 1 mol NCO Gruppen umgesetzt waren, wurde der Katalysator durch Zugabe einer zum Katalysator äquivalenten Menge an p-Toluolsulfonsäure (als 40%ige Lösung in Isopropanol) deaktiviert, weitere 30 min bei Reaktionstemperatur nachgerührt und anschließend aufgearbeitet.

Die Aufarbeitung der Rohlösung, deren $n_D^{20}$ 1,4610 betrug, erfolgte durch Vakuumdestillation in einem Dünnschichtverdampfer, Typ Kurzwegverdampfer (KWV), mit vorgeschaltetem Vorverdampfer (VV) (Destillationsbedingungen: Druck: 0,08 +/- 0,04 mbar, VV-Temperatur: 120°C, KWV-Temp.: 140°C), wobei nicht umgesetztes Monomer als Destillat und das monomerenarme Polyisocyanatharz als Sumpfprodukt separiert wurden (Startdurchlauf: Beispiel 1-A). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde verfahren wie eingangs beschrieben, mit dem Unterschied, dass der Isocyanatumsatz (indiziert durch den Brechungsindex der Rohware) von Durchlauf zu Durchlauf stufenweise angehoben wurde. Diese Verfahrensweise wurde mehrmals wiederholt. Die Ergebnisse sind Tabelle 1 zu entnehmen.

Tabelle 1

| Bsp. 1- | Bu$_4$P$^+$ [HF$_2$]$^-$ Lösung.[a] [mg] | n$_D^{20}$ [b] | Delta-n$_D^{20}$ [c] | Harzmenge [g] | Harz-NCO-Gehalt [%] | Iminooxadiazindione[d] | Isocyanurate[d] | Uretdione[d] |
|---|---|---|---|---|---|---|---|---|
| A | 507 | 1,4610 | 0,0087 | 195 | 23,6 | 44,3% | 50,5% | 5,0% |
| B | 340 | 1,4604 | 0,0081 | 198 | 23,5 | 49,4% | 47,1% | 3,3% |
| C | 402 | 1,4647 | 0,0124 | 251 | 23,7 | 43,0% | 51,0% | 5,7% |
| D | 379 | 1,4650 | 0,0127 | 261 | 23,1 | 44,6% | 51,4% | 3,8% |
| E | 402 | 1,4665 | 0,0142 | 290 | 22,9 | 42,9% | 51,8% | 5,0% |
| F | 424 | 1,4668 | 0,0145 | 296 | 23,1 | 43,0% | 52,6% | 4,0% |
| G | 469 | 1,4678 | 0,0155 | 310 | 23,0 | 40,0% | 53,2% | 4,8% |
| H | 491 | 1,4680 | 0,0157 | 317 | 22,9 | 42,5% | 50,1% | 4,7% |
| I | 580 | 1,4740 | 0,0217 | 434 | 22,3 | 39,5% | 55,3% | 4,7% |
| J | 670 | 1,4745 | 0,0222 | 443 | 21,4 | 38,9% | 55,6% | 4,1% |
| K | 804 | 1,4805 | 0,0282 | 535 | 21,0 | 37,9% | 57,9% | 4,1% |
| L | 1216 | 1,4852 | 0,0329 | 608 | 20,5 | 36,2% | 57,6% | 3,9% |
| M | 1183 | 1,4920 | 0,0397 | 696 | 19,2 | 31,5% | 63,8% | 2,8% |

[a] 70%ig in iPrOH; [b] Brechungsindex der Reaktionsmischung nach Einwirkung des Stoppers vor der Destillation, [c] Anstieg des Brechungsindex gg. Ausgangswert vor erster Katalysatorzugabe, [d] mol-% lt. NMR, bezogen auf die Summe der in der Modifizierungsreaktion gebildeten NCO-Folgeprodukte, Differenz zu 100%: Urethane/ Allophanate

**Beispiel 2 erfindungsgemäß**

Additiv: Isoeicosan (relative Permittivität bei 25 °C/ 50 Hz: 2,1)

Es wurde verfahren wie in Bsp. 1 beschrieben, mit dem Unterschied, dass dem entgasten HDI 20 % Isoeicosan zugesetzt wurden. Da Isoeicosan eine dem HDI vergleichbare Flüchtigkeit aufweist, wurde bei der Aufarbeitung verfahren wie in Bsp. 1beschrieben.

Tabelle 2

| Bsp. 2 | Bu$_4$P$^+$[HF$_2$]$^-$-Lösung[a] [mg] | Delta-n$_D$ [b] | Harzmenge [g] | Harz-NCO [%] | Iminooxadiazindione[c] | Iso-cyanurate[c] | Uretdione[c] |
|---|---|---|---|---|---|---|---|
| A | 720 | 0,0080 | 209 | 23,4 | 54,6% | 41,7% | 3,2% |
| B | 670 | 0,0078 | 220 | 23,2 | 56,6% | 38,9% | 3,6% |
| C | 692 | 0,0077 | 228 | 23,3 | 53,7% | 39,6% | 3,1% |
| D | 692 | 0,0162 | 377 | 22,0 | 53,6% | 43,2% | 3,1% |
| E | 714 | 0,0173 | 391 | 21,7 | 51,3% | 44,0% | 3,6% |
| F | 714 | 0,0218 | 513 | 21,3 | 50,5% | 46,3% | 3,1% |
| G | 893 | 0,0217 | 498 | 21,0 | 49,2% | 47,3% | 3,1% |
| H | 982 | 0,0258 | 621 | 20,3 | 47,7% | 49,5% | 2,6% |
| I | 1004 | 0,0264 | 611 | 20,2 | 45,4% | 51,0% | 2,9% |
| J | 1071 | 0,0296 | 657 | 19,5 | 45,5% | 52,3% | 2,0% |
| K | 1071 | 0,0286 | 658 | 20,0 | 45,1% | 51,9% | 2,4% |
| L | 1116 | 0,0290 | 696 | 18,9 | 42,6% | 54,3% | 2,1% |
| M | 1205 | 0,0299 | 694 | 18,4 | 42,9% | 54,0% | 2,2% |
| N | 1272 | 0,0325 | 732 | 17,9 | 40,1% | 54,5% | 2,2% |
| O | 1272 | 0,0386 | 902 | 15,9 | 31,5% | 60,4% | 1,3% |

[a] 70%ig in iPrOH; [b] Anstieg des Brechungsindex gg. Ausgangswert vor erster Katalysatorzugabe, hier wurde bei 60°C gemessen, da die Reaktionsmischungen ab einem gewissen Umsatz bei 20°C heterogen wurden und eine Bestimmung des n$_D^{20}$ entweder nicht möglich war oder irreführende Werte lieferte [d] mol-% lt. NMR, bezogen auf die Summe der in der Modifizierungsreaktion gebildeten NCO-Folgeprodukte, Differenz zu 100%: Urethane/Allophanate

**Beispiel 3 erfindungsgemäß**

Additiv: n-Hexan (relative Permittivität bei 25 °C/ 50 Hz: 1,9)

Es wurde verfahren wie in Beispiel 1 beschrieben, mit dem Unterschied, dass dem entgasten HDI 20 % n-Hexan zugesetzt wurden und dass das n-Hexan nach der jeweiligen Reaktion und vor der Vakuumdestillation durch Passage durch den auf 120°C (VV) und 140°C (KWV) geheizten Destillationsapparat bei Normaldruck abgetrennt und in den nächsten Ansatz dosiert wurde. Anschließend erfolgte die destillative Auftrennung von Recyclatmonomer und Polyisocyanatharz durch Vakuumdestillation wie in Bsp. 1 beschrieben.

Tabelle 3

| Bsp. 3 | Bu$_4$P$^+$[HF$_2$]$^-$-Lösung[a] [mg] | Delta-n$_D$ [b] | Harzmenge [g] | Harz-NCO [%] | Iminooxadiazindione[c] | Iso-cyanurate[c] | Uretdione[c] |
|---|---|---|---|---|---|---|---|
| A | 660 | 0,0075 | 205 | 23,7 | 57,2% | 39,5% | 3,2% |

(fortgesetzt)

| Bsp. 3 | Bu$_4$P$^+$[HF$_2$]$^-$ -Lösung[a] [mg] | Delta-n$_D$ [b] | Harzmenge [g] | Harz-NCO [%] | Iminooxadiazindione[c] | Iso-cyanurate[c] | Uretdione[c] |
|---|---|---|---|---|---|---|---|
| B | 650 | 0,0072 | 198 | 23,4 | 58,4% | 38,5% | 2,8% |
| C | 682 | 0,0158 | 390 | 21,8 | 50,8% | 43,8% | 4,2% |
| D | 698 | 0,0210 | 502 | 20,8 | 49,3% | 47,5% | 2,9% |
| E | 915 | 0,0260 | 600 | 19,8 | 46,5% | 48,9% | 3,0% |
| F | 950 | 0,0285 | 668 | 19,8 | 44,2% | 52,3% | 2,9% |
| G | 1180 | 0,0315 | 730 | 18,1 | 39,5% | 54,9% | 2,4% |
| H | 1302 | 0,0392 | 915 | 15,4 | 33,3% | 62,3% | 1,8% |

[a] 70%ig in iPrOH; [b] Anstieg des Brechungsindex gg. Ausgangswert vor erster Katalysatorzugabe, hier wurde bei 60°C gemessen, da die Reaktionsmischungen ab einem gewissen Umsatz bei 20°C heterogen wurden und eine Bestimmung des $n_D^{20}$ entweder nicht möglich war oder irreführende Werte lieferte [d] mol-% lt. NMR, bezogen auf die Summe der in der Modifizierungsreaktion gebildeten NCO-Folgeprodukte, Differenz zu 100%: Urethane/Allophanate

Bei einem Vergleich der in Tabellen 2 und 3 aufgeführten Daten mit denjenigen aus Tabelle 1 ist ersichtlich, dass bei vergleichbarem Monomerumsatz durch Einsatz der unpolaren Additive Isoeicosan oder auch n-Hexan eine deutliche Erhöhung des Iminooxadiazindiongruppenanteils in der Polyisocyanatharzen erzielt wird.

## Patentansprüche

1. Verfahren zur Herstellung von Iminooxadiazindiongruppen enthaltenden Polyisocyanaten, bei dem mindestens ein monomeres Di- und/oder Triisocyanat, in Gegenwart von

a) mindestens einem Katalysator,
b) mindestens einem Additiv (A) mit einer relativen Permittivität bei 18 °C bis 30°C von weniger als 4,0, bestimmt mit derjenigen Messmethode wie sie in der Beschreibung angegeben ist,
c) gegebenenfalls weiteren, von A verschiedenen Additiven

oligomerisiert wird, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ([F$^-$ × HF)$_m$]), wobei m einen Zahlenwert von 0,001 bis 2,000 besitzt.

2. Verfahren nach Anspruch 1, **dadurch gekennzeichnet, dass** dem monomeren Di- und/oder Triisocyanat das Additiv (A) vor dem Inkontaktbringen mit dem Katalysator zugesetzt wird.

3. Verfahren nach Anspruch 1 oder 2, **dadurch gekennzeichnet, dass** als Additiv (A) ein lineares oder verzweigtes, aliphatisches, cycloaliphatisches und/oder araliphatisches C$_4$-C$_{30}$-Alkan, insbesondere C$_5$-C$_{30}$-Alkan, oder Mischungen davon eingesetzt werden, wobei das Additiv (A) bevorzugt ausgewählt ist aus der Gruppe umfassend lineares, verzweigtes oder cyclisches Butan, Pentan, Hexan, Heptan, Octan, Nonan, Decan, Undecan, Dodecan, Tridecan, Tetradecan, Pentadecan, Hexadecan, Heptadecan, Octadecan, Nonadecan, Eicosan, Heneicosan, Docosan, Tricosan, -Tetracosan, Pentacosan, Hexacosan, Heptacosan, Octacosan, Nonacosan, Tricontan, sowie deren Mischungen.

4. Verfahren nach einem der vorstehenden Ansprüche, **dadurch gekennzeichnet, dass** 1 bis 50 Gew.-%, bevorzugt 2 bis 30 Gew.-%, besonders bevorzugt 2 bis 20 Gew.-% an Additiv (A), bezogen auf die Masse des monomeren Di- und/oder Triisocyanats eingesetzt werden.

5. Verfahren nach einem der vorstehenden Ansprüche, **dadurch gekennzeichnet, dass** das Additiv (A) eine relative Permittivität bei 18 °C bis 30 °C von höchstens 3,5 aufweist, bevorzugt von höchstens 3,0, besonders bevorzugt von höchstens 2,8 oder gar höchstens 2,5.

6. Verfahren nach einem der vorstehenden Ansprüche, **dadurch gekennzeichnet, dass** als monomeres Di- und/oder Triisocyanat ein aliphatisches Diisocyanat eingesetzt wird, insbesondere Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat und/oder 4-Isocyanatomethyl-1,8-octandiisocyanat, bevorzugt HDI.

7. Verfahren nach einem der vorstehenden Ansprüche, **dadurch gekennzeichnet, dass** bei den Di- und/oder Poly(hydrogen)fluoriden ($[F^- \times HF_m]$) m einen Zahlenwert von 0,1 bis 2,000 besitzt, bevorzugt 0,5 bis 2,000.

8. Verfahren nach einem der vorstehenden Ansprüche, **dadurch gekennzeichnet, dass** es sich bei dem Di- und/oder Poly(hydrogen)fluorid ($[F^- \times HF_m]$) um ein quaternäres Ammoniumdifluorid, ein Phosphoniumdifluorid, handelt, vorzugsweise um solche, wie sie durch Abmischen von quaternären Ammonium- sowie Phosphonium-fluoriden oder -hydroxiden mit entsprechenden Mengen, optional in Alkoholen oder Wasser vorgelöstem Fluorwasserstoff bereitet werden können.

9. Verfahren nach einem der vorstehenden Ansprüche, **dadurch gekennzeichnet, dass** der Katalysator/die Katalysatormischung in einem Anteil von 1 mol-ppm bis 1 mol-%, bevorzugt 5 mol-ppm bis 0,1 mol-%, jeweils bezogen auf die Menge an monomerem Di- und/oder Triisocyanat eingesetzt wird.

10. Verfahren nach einem der vorstehenden Ansprüche, **dadurch gekennzeichnet, dass** das Verfahren im Temperaturbereich von 0 °C bis + 250 °C durchgeführt wird, bevorzugt 20 bis 180 °C, besonders bevorzugt 40 bis 150 °C.

11. Verfahren nach einem der vorstehenden Ansprüche, **dadurch gekennzeichnet, dass** die Oligomerisierung abgebrochen wird, nachdem 5 bis 80 Gew.-% des eingesetzten monomeren Di- und/oder Triisocyanates umgesetzt sind, bevorzugt 10 bis 60 Gew.-%.

12. Verfahren nach Anspruch 11, **dadurch gekennzeichnet, dass** die Oligomerisierung abgebrochen wird, indem der Katalysator desaktiviert wird, insbesondere durch Zugabe einer Säure oder eines Säurederivates wie Benzoylchlorid, eines sauren Esters Phosphor oder Schwefel enthaltender Säuren, diese Säuren selbst, adsorptive Bindung des Katalysators und anschließende Abtrennung durch Filtration oder Kombinationen hiervon.

13. Verfahren nach Anspruch 11 oder 12, **dadurch gekennzeichnet, dass** nicht umgesetztes Monomer aus der Reaktionsmischung abgetrennt wird.

14. Reaktionssystem zur Herstellung von Iminooxadiazindiongruppen enthaltenden Polyisocyanaten, umfassend mindestens ein monomeres Di- und/oder Triisocyanat, sowie

    a) mindestens einem Katalysator,
    b) mindestens ein Additiv (A) mit einer relativen Permittivität bei 18 °C bis 30 °C von weniger als 4,0, bestimmt mit derjenigen Messmethode wie sie in der Beschreibung angegeben ist,
    c) gegebenenfalls weitere, von A verschiedene Additive,

wobei als Katalysator ein Tetraorganyl-ammoniumsalz und/oder - phosphoniumsalz eingesetzt wird und wobei die Anionen des Tetraorganyl-ammoniumsalzes und/oder - phosphoniumsalzes ausgewählt sind aus der Gruppe Di- und/oder Poly(hydrogen)fluoride ($[F^- \times HF_m]$), wobei m einen Zahlenwert von 0,001 bis 2,000 besitzt.

15. Verwendung von Verbindungen mit einer relativen Permittivität bei 18 °C bis 30 °C von weniger als 4,0, bestimmt mit derjenigen Messmethode wie sie in der Beschreibung angegeben ist, als Additiv (A) zur Herstellung von Iminooxadiazindiongruppen enthaltenden Polyisocyanaten durch katalysierte Modifizierung monomerer Di- und/oder Triisocyanate, wobei als Katalysator ein Tetraorganyl-ammoniumsalz und/oder - phosphoniumsalz eingesetzt wird und wobei die Anionen des Tetraorganyl-ammoniumsalzes und/oder -phosphoniumsalzes ausgewählt sind aus der Gruppe Di- und/oder Poly(hydrogen)fluoride ($[F^- \times HF_m]$), wobei m einen Zahlenwert von 0,001 bis 2,000 besitzt.

**Claims**

1. Process for preparing polyisocyanates containing iminooxadiazinedione groups, by oligomerizing at least one monomeric di- and/or triisocyanate in the presence of

   a) at least one catalyst,
   b) at least one additive (A) having a relative permittivity at 18°C to 30°C of less than 4.0, determined by the measurement method specified in the description,
   c) optionally further additives different from A,

   where the catalyst used comprises a tetraorganylammonium salt and/or tetraorganylphosphonium salt and where the anions of the tetraorganylammonium salt and/or tetraorganylphosphonium salt are selected from the group of di- and/or poly(hydrogen)fluorides ($[F^- \times HF]_m$]), where m possesses a numerical value of 0.001 to 2.000.

2. Process according to Claim 1, **characterized in that** the additive (A) is added to the monomeric di- and/or triisocyanate before the latter is brought into contact with the catalyst.

3. Process according to Claim 1 or 2, **characterized in that** the additive (A) used comprises a linear or branched, aliphatic, cycloalphatic and/or araliphatic $C_4$-$C_{30}$ alkane, especially $C_5$-$C_{30}$ alkane, or mixtures thereof, the additive (A) being preferably selected from the group encompassing linear, branched or cyclic butane, pentane, hexane, heptane, octane, nonane, decane, undecane, dodecane, tridecane, tetradecane, pentadecane, hexadecane, heptadecane, octadecane, nonadecane, eicosane, heneicosane, docosane, tricosane, tetracosane, pentacosane, hexacosane, heptacosane, octacosane, nonacosane, tricontane, and also mixtures thereof.

4. Process according to any of the preceding claims, **characterized in that** 1 to 50 wt%, preferably 2 to 30 wt%, more preferably 2 to 20 wt% of additive (A) is used, based on the mass of the monomeric di- and/or triisocyanate.

5. Process according to any of the preceding claims, **characterized in that** the additive (A) has a relative permittivity at 18°C to 30°C of at most 3.5, preferably of at most 3.0, more preferably of at most 2.8 or even at most 2.5.

6. Process according to any of the preceding claims, **characterized in that** the monomeric di- and/or triisocyanate used comprises an aliphatic diisocyanate, more particularly hexamethylene diisocyanate (HDI), 2-methylpentane 1,5-diisocyanate, 2,4,4-trimethylhexane 1,6-diisocyanate, 2,2,4-trimethylhexane 1,6-diisocyanate and/or 4-isocyanatomethyloctane 1,8-diisocyanate, preferably HDI.

7. Process according to any of the preceding claims, **characterized in that** for the di- and/or poly(hydrogen) fluorides ($[F^- \times HF]_m$]) m possesses a numerical value of 0.1 to 2.000, preferably 0.5 to 2.000.

8. Process according to any of the preceding claims, **characterized in that** the di- and/or poly(hydrogen) fluoride ($[F^- \times HF]_m$]) is a quaternary ammonium difluoride or a phosphonium difluoride, preferably of the kind preparable by blending quaternary ammonium and phosphonium fluorides or hydroxides with corresponding amounts of hydrogen fluoride, optionally dissolved beforehand in alcohols or water.

9. Process according to any of the preceding claims, **characterized in that** the catalyst/the catalyst mixture is used in a fraction of 1 mol-ppm to 1 mol%, preferably 5 mol-ppm to 0.1 mol%, based in each case on the amount of monomeric di- and/or triisocyanate.

10. Process according to any of the preceding claims, **characterized in that** the process is carried out in the temperature range from 0°C to +250°C, preferably 20 to 180°C, more preferably 40 to 150°C.

11. Process according to any of the preceding claims, **characterized in that** the oligomerization is terminated after reaction of 5 to 80 wt% of the monomeric di- and/or triisocyanate used, preferably 10 to 60 wt%.

12. Process according to Claim 11, **characterized in that** the oligomerization is terminated by deactivating the catalyst, more particularly by adding an acid or an acid derivative such a benzoyl chloride, an acidic ester of phosphorus-containing or sulfur-containing acids, these acids themselves, adsorptive binding of the catalyst and subsequent removal by filtration, or combinations thereof.

13. Process according to Claim 11 or 12, **characterized in that** unreacted monomer is removed from the reaction mixture.

14. Reaction system for preparing polyisocyanates containing iminooxadiazinedione groups, comprising at least one monomeric di- and/or triisocyanate and also

a) at least one catalyst,
b) at least one additive (A) having a relative permittivity at 18°C to 30°C of less than 4.0, determined by the measurement method specified in the description,
c) optionally further additives different from A,

where the catalyst used comprises a tetraorganylammonium salt and/or tetraorganylphosphonium salt and where the anions of the tetraorganylammonium salt and/or tetraorganylphosphonium salt are selected from the group of di- and/or poly(hydrogen)fluorides ($[F^- \times HF]_m$), where m possesses a numerical value of 0.001 to 2.000.

15. Use of compounds having a relative permittivity at 18°C to 30°C of less than 4.0, determined by the measurement method as specified in the description, as additive (A) for preparing polyisocyanates containing iminooxadiazinedione groups by catalyzed modification of monomeric di- and/or triisocyanates, where the catalyst used comprises a tetraorganylammonium salt and/or tetraorganylphosphonium salt and where the anions of the tetraorganylammonium salt and/or tetraorganylphosphonium salt are selected from the group of di- and/or poly(hydrogen)fluorides ($[F^- \times HF]_m$), where m possesses a numerical value of 0.001 to 2.000.

**Revendications**

1. Procédé de préparation de polyisocyanates contenant des groupes iminooxadiazinedione, dans lequel on oligomérise au moins un di- et/ou un triisocyanate monomère, en présence

a) d'au moins un catalyseur,
b) d'au moins un additif (A) ayant une permittivité relative, à 18 °C à 30 °C, inférieure à 4,0, déterminée par la méthode de mesure telle qu'indiquée dans la description,
c) éventuellement d'autres additifs différents de A,

dans lequel on utilise en tant que catalyseur un sel de tétraorganylammonium et/ou de tétraorganylphosphonium, et dans lequel les anions du sel de tétraorganylammonium et/ou de tétraorganylphosphonium sont choisis dans le groupe consistant en les di- et/ou les poly(fluorures (d'hydrogène)) ($[F^- \times HF)_m]$), m ayant une valeur numérique de 0,001 à 2,000.

2. Procédé selon la revendication 1, **caractérisé en ce qu'**on ajoute au di- et/ou au triisocyanate monomère l'additif (A) avant la mise en contact avec le catalyseur.

3. Procédé selon la revendication 1 ou 2, **caractérisé en ce qu'**on utilise en tant qu'additif (A) un alcane en $C_4$-$C_{30}$, en particulier un alcane en $C_5$-$C_{30}$, linéaire ou ramifié, aliphatique, cycloaliphatique et/ou araliphatique, ou des mélanges de ceux-ci, l'additif (A) étant de préférence choisi dans le groupe comprenant les composés linéaires, ramifiés ou cycliques butane, pentane, hexane, heptane, octane, nonane, décane, undécane, dodécane, tridécane, tétradécane, pentadécane, hexadécane, heptadécane, octadécane, nonadécane, éicosane, hénéicosane, docosane, tricosane, tétracosane, pentacosane, hexacosane, heptacosane, octacosane, nonacosane, tricontane, ainsi que leurs mélanges.

4. Procédé selon l'une des revendications précédentes, **caractérisé en ce qu'**on utilise 1 à 50 % en poids, de préférence 2 à 30 % en poids, d'une manière particulièrement préférée 2 à 20 % en poids de l'additif (A), par rapport à la masse du di- et/ou du triisocyanate monomère.

5. Procédé selon l'une des revendications précédentes, **caractérisé en ce que** l'additif (A) présente une permittivité relative, à 18 °C à 30 °C, d'au plus 3,5, de préférence d'au plus 3,0, d'une manière particulièrement préférée d'au plus 2,8, ou même d'au plus 2,5.

6. Procédé selon l'une des revendications précédentes, **caractérisé en ce qu'**on utilise en tant que di- et/ou triisocyanate monomère un diisocyanate aliphatique, en particulier le diisocyanate d'hexaméthylène (HDI), le 1,5-diisocyanato-2-méthylpentane, le 1,6-diisocyanato-2,4,4-triméthyl-hexane, le 1,6-diisocyanato-2,2,4-triméthyl-hexane et/ou le 1,8-diisocyanato-4-isocyanatométhyl-octane, de préférence le HDI.

7. Procédé selon l'une des revendications précédentes, **caractérisé en ce que**, dans les di- et/ou poly(fluorures (d'hydrogène)) ($[F^- \times HF]_m$]), m a une valeur numérique de 0,1 à 2 000, de préférence de 0,5 à 2 000.

8. Procédé selon l'une des revendications précédentes, **caractérisé en ce que**, pour ce qui concerne le di- et/ou le poly(fluorure (d'hydrogène)) ($[F^- \times HF]_m$]), il s'agit d'un difluorure d'ammonium quaternaire, d'un difluorure de phosphonium, de préférence de ceux que l'on peut préparer par mélange de fluorures ou d'hydroxydes d'ammonium quaternaire, ainsi que de phosphonium, avec des quantités appropriées d'un fluorure d'hydrogène éventuellement dissous au préalable dans des alcools ou de l'eau.

9. Procédé selon l'une des revendications précédentes, **caractérisé en ce que** le catalyseur/le mélange de catalyseurs est utilisé selon une proportion de 1 ppm en moles à 1 % en moles, de préférence de 5 ppm en moles à 0,1 % en moles, dans chaque cas par rapport à la quantité du di- et/ou du triisocyanate monomère.

10. Procédé selon l'une des revendications précédentes, **caractérisé en ce que** le procédé est mis en œuvre dans la plage de températures de 0 °C à + 250 °C, de préférence de 20 à 180 °C, d'une manière particulièrement préférée de 40 à 150 °C.

11. Procédé selon l'une des revendications précédentes, **caractérisé en ce que** l'oligomérisation est interrompue après que 5 à 80 % en poids du di- et/ou du triisocyanate monomère utilisé ont réagi, de préférence 10 à 60 % en poids.

12. Procédé selon la revendication 11, **caractérisé en ce que** l'oligomérisation est interrompue par désactivation du catalyseur, en particulier par addition d'un acide ou d'un dérivé d'un acide tel que le chlorure de benzoyle, d'un ester acide d'acides phosphorés ou sulfurés, de ces acides proprement dits, par liaison du catalyseur puis séparation par filtration, ou des combinaisons de ces techniques.

13. Procédé selon la revendication 11 ou 12, caractérisé en que le monomère n'ayant pas réagi est séparé du mélange réactionnel.

14. Système réactionnel pour la préparation de polyisocyanates contenant des groupes iminooxadiazinedione, comprenant au moins un di- et/ou un triisocyanate monomère, ainsi que

    a) au moins un catalyseur,
    b) au moins un additif (A) ayant une permittivité relative, à 18 °C à 30 °C, inférieure à 4,0, déterminée par la méthode de mesure telle qu'indiquée dans la description,
    c) éventuellement d'autres additifs différents de A,

dans lequel on utilise en tant que catalyseur un sel de tétraorganylammonium et/ou un sel de tétraorganylphosphonium, et les anions du sel de tétraorganylammonium et/ou de tétraorganylphosphonium étant choisis dans le groupe consistant en les di- et/ou les poly(fluorures (d'hydrogène)) ($[F^- \times HF]_m$]), m ayant une valeur numérique de 0,001 à 2,000.

15. Utilisation de composés ayant une permittivité relative, à 18 °C à 30 °C, inférieure à 4,0, déterminée par la méthode de mesure telle qu'indiquée dans la description, en tant qu'additif (A) pour la préparation de polyisocyanates contenant des groupes iminooxadiazinedione par modification catalysée de di- et/ou de triisocyanates monomères, pour laquelle on utilise en tant que catalyseur un sel de tétraorganylammonium et/ou un sel de tétraorganylphosphonium, et les anions du sel de tétraorganylammonium et/ou du sel de tétraorganylphosphonium étant choisis dans le groupe consistant en les di- et/ou les poly(fluorures (d'hydrogène)) ($[F^- \times HF]_m$]), m ayantune valeur numérique de 0,001 à 2,000.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 962455 A1
- EP 962454 A1
- EP 896009 A1
- EP 798299 A1
- EP 447074 A1
- EP 379914 A1
- EP 339396 A1

- EP 315692 A1
- EP 295926 A1
- EP 235388 A1
- EP 0962455 A1
- EP 896009 A
- EP 962454 A

**In der Beschreibung aufgeführte Nicht-Patentliteratur**

- **H. J. LAAS et al.** *J. Prakt. Chem.,* 1994, vol. 336, 185

- **D. WENDISCH ; H. REIFF ; D. DIETERICH.** *Die Angewandte Makromolekulare Chemie,* 1986, vol. 141, 173-183